# EUROPEAN PATENT SPECIFICATION

(11) **EP 3 186 833 B1**
(45) Date of publication and mention of the grant of the patent: **22.08.2018**
(21) Application number: 15725591.0
(22) Date of filing: 26.05.2015
(51) Int. Cl.: H01L 27/146

(54) **RADIATION DETECTOR DEVICE**
STRAHLUNGSDETEKTOR
DISPOSITIF DE DÉTECTION DE RAYONNEMENT

(43) Date of publication of application: 05.07.2017
(73) Proprietor: Siemens Healthcare GmbH, 91052 Erlangen (DE)
(72) Inventor: LABAYEN DE INZA, Miguel, 91301 Forchheim (DE); WREGE, Jan, 91056 Erlangen (DE)
(86) International application number: PCT/EP2015/061548
(87) International publication number: WO 2016/188561

(56) References cited:
- WO-A2-03/077319
- GB-A- 2 319 394
- US-A- 5 328 660
- HEIKKINEN H ET AL: "Indium-tin bump deposition for the hybridization of CdTe sensors and readout chips", NUCLEAR SCIENCE SYMPOSIUM CONFERENCE RECORD (NSS/MIC), 2010 IEEE, IEEE, 30 October 2010 (2010-10-30), pages 3891-3895, XP032054819, DOI: 10.1109/NSSMIC.2010.5874544 ISBN: 978-1-4244-9106-3

## Description

The invention relates to a radiation detector device, a medical imaging device and a method for producing a radiation detector device. The radiation detector device of the invention is a photon counting radiation detector device that is particularly suitable for medical applications with strong mechanical requirements, e.g. CT scanners. For the connection of the detector substrate and the readout substrate, a solder alloy comprising Bismuth, Tin and Silver is used.

A photon counting radiation detector device is capable of direct conversion of the energy of the ionizing radiation to electrical charge. These radiation detector devices typically comprise an extended semiconductor material, for example CdTe, CdZnTe (CZT), CdZnTeSe, CdTeSe, CdMnTe, InP, TlBr2, or HgI2, as detector substrate. The energy of the impinging ionizing radiation is converted directly to electrical charges. The charges are separated by an electric field between the charge biasing electrode (cathode) and the readout contact (anode). The charge collector contact and the readout contact are connected by a connection comprising a solder connection. The electrical charge induces a signal at the input of the readout substrate (e.g. ASIC). The ASIC is connected to peripheral electronics, e.g. via wirebond connection or through silicon vias.

Medical imaging devices like diagnostic devices for fluoroscopy or normal radiography, X-ray systems with a C-arm or Computed Tomography scanners (CT scanners) are well known. The mechanical requirements of standard radiography equipment, fluoroscopy etc., are very relaxed due to the static nature of the equipment and the measurement. However, X-ray based counting systems with C-arm and in greater degree CT scanners have elevated mechanical requirements for the radiation detector device due to the movement of the radiation detector device which is mounted on the C-arm or, in the case of a CT scanner, the elevated g-forces due to the rotation of the gantry. Moreover, the mechanical stresses due to the elevated rotational g-forces are greatly amplified by the combination of the thermo-mechanical stresses due to the on-off power cycles (thermal cycling) during the CT operation. Photon counting detector devices comprising a semiconductor material which contains Cadmium, e.g. CdTe or CdZnTe, have elevated properties, like an appropriate detection efficiency, over other semiconductor materials in the field of medical imaging. Besides thermo-mechanical stress due to the thermal cycling, semiconductor materials comprising Cadmium, like CdTe or CdZnTe (CZT) etc., are temperature sensitive and suffer a strong deterioration of their X-ray or gamma detecting properties when exposed to temperatures above 180°C. Therefore, they require a low temperature solder for bonding of the detector substrate and the readout substrate. In conclusion, a solder is needed which allows the use of the radiation detector device under strong mechanical requirements, which offers a low melting temperature and which withstands elevated g-forces in combination with thermal cycling, and which for environmental reasons should be a lead-free solder material.
The document IEEE Electron. Compon. And Technol. Conf. 1998 pp. 277-283 by Fay Hua, Zequn Mei and Judy Glazer (doi: 10.1109/ECTC.1998.678706), discloses an eutectic SnBi as an alternative Pb-free solder. The eutectic 58Bi-42Sn solder is disclosed as a potential alternative for 63Sn-37Pb solder in the applications of consumer products. Heikkinen et al. shows in the article "Indium-Tin bump deposition for the hybridization of CdTe sensors and readout chips" (Nuclear Science Symposium Conference Record, 2010 IEEE, 30 October 2010, pages 3891-3895) a low temperature bump bonding process to assemble CdTe sensors to readout chips. US6933505 relates to a bump-bonded radiation imaging device wherein a low temperature binary SnBi alloy is used for bump bonding.
However, the binary alloy SnBi is not very suitable for use within devices experiencing thermal and mechanical stresses comprising a photon counting detector, such as a CT scanner, due to its poor mechanical and thermo-mechanical performance.

US5368814A1 and EP629467A1 relate to lead free, Tin-Bismuth solder alloys. The disclosed solder alloys are particularly useful in joining integrated circuit chips to chip carriers and substrates, as printed circuit boards, joining chip carriers to substrates, and joining circuitization lands and pads in multilayer printed circuit boards.

US5569433 relates to a lead-free low melting solder with improved mechanical properties. The solder alloys can be used in the manufacture of articles, e.g. surface mounted circuit boards or laser chips solder-bonded to a sub-mount.

However, the above alloys fail to provide a solder which provides mechanical and thermo-mechanical properties required by a detector substrate comprising Cadmium and its use within a X-ray or gamma ray counting radiation detector device under strong mechanical requirements like present in a CT scanner.

It is a primary object of the invention to provide a radiation detector device which is useful for medical applications with strong mechanical requirements.

It is a further object of the invention to provide a radiation detector device comprising a temperature sensitive detector substrate and a solder connection with adequate mechanical and thermo-mechanical stability under strong mechanical conditions.

It is a further object of the invention to provide a medical imaging device comprising said radiation detector device and a method for producing said radiation detector device.

In order to solve the above problem and accomplish the object, there is provided a radiation detector device according to claim 1, a medical imaging device according to claim 12 and a method for producing a radiation detector device according to claim 14.

The invention relates to a radiation detector device comprising a detector substrate for direct conversion of X-ray and/or gamma quanta to electrical charge, wherein the detector substrate comprises a semiconductor material comprising Cadmium, a readout substrate having a readout contact, a charge collector contact for collecting an electrical charge from said detector substrate, and a connection wherein the connection electrically interconnects the charge collector contact and the readout contact, wherein the connection comprises a solder connection wherein the solder comprises Bismuth, Tin and Silver.

The radiation detector device comprises at least one detection unit or pixel which comprises a volume of the detector substrate wherein the volume is defined by the electric field of a bias voltage, the charge collector contact, the connection between the charge collector contact and the readout contact, the readout contact and the readout substrate offering signal processing of said pixel. The radiation detector device may comprise a pixilated structure which provides a plurality of detection units or pixels. The number of pixels may be as large as 1,000,000 or even larger. A plurality of radiation detector devices may be tiled to form a detector module or detector array. The radiation detector device may be embodied as a hybrid assembly comprising a single detector substrate and a single readout substrate. In a further embodiment, the radiation detector device may be constructed as a hybrid assembly comprising a plurality of detector substrates and/or readout substrates.

During operation, the detector substrate commonly faces a radiation source, a cover or other components in front of the detector substrate may be present. The detector substrate comprises a charge biasing electrode on the front side of the detector substrate. The detector substrate further comprises a charge collector contact which can be part of the charge collector electrode and which is located at the back side of the detector substrate. An electric biasing field can be applied between the readout contact and the charge biasing electrode in order to separate charges induced by energy deposition in the detector substrate, e.g. by a photon.

The detector substrate comprises a semiconductor material which contains Cadmium which is beneficial for the detection of X-ray or gamma ray photons in imaging applications, in particular medical imaging applications.

The readout substrate has a readout contact (e.g. a standard metallization readout contact). The readout substrate may be used and/or configured to provide analog and digital processing of the signals. The resulting signals can be directed to further data processing units. The radiation detector device can be controlled by a peripheral controller unit.

The connection between the detector substrate and the readout substrate comprises a solder connection. The solder connection comprises a solder. The solder comprises Tin, Bismuth and Silver. The solder mixture can be abbreviated as SnBiAg. For example, the solder can be used as prefabricated alloy, e.g. in the form of a solder ball or solder paste. In a further embodiment, the solder material can also be used in combination with copper pillars. Besides providing electrical conductive connection between the detector substrate and the readout substrate, the connection serves to transport heat from the detector substrate towards the readout substrate and other components.

The inclusion of small amounts of Silver in a solder comprising Bismuth and Tin results in similar material properties like density, coefficient of thermal expansion and melting temperature, but at the same time an improvement of thermal conductivity and reliability of the solder connection or solder joints is obtained. Therefore a longer lifetime of the radiation detector device may be achieved. It is to be understood that silver must be present in measureable amounts and not merely as trace metal impurities.

The invention further relates to a medical imaging device comprising a radiation detector device.

The medical imaging device comprises the radiation detector device. The medical imaging device uses X-rays and/or gamma rays for imaging purposes. Due to the detector substrate comprising Cadmium and the solder connection comprising SnBiAg, the medical imaging device benefits from the advantages given for the radiation detector device like an improved thermo-mechanical stability over other solders like binary SnBi and a longer lifetime of the radiation detector device.

The invention relates to a method for producing a radiation detector device comprising a plurality of steps. In a first step, a readout substrate is provided wherein the readout substrate has a readout contact. In another step, a solder connection is applied wherein the solder connection contains a solder comprising Bismuth, Tin and Silver. In another step, the detector substrate is disposed to bond charge collector contact and readout contact by a solder reflow technique to form an electrically conductive interconnection of corresponding charge collector contact and readout contact.

The readout substrate is provided. To be prepared for the application of the solder connection, a readout contact, e.g. a standard metallization readout contact, is provided.

In the next step, the solder is applied, e.g. in the form of a solder ball or solder paste. In an alternative embodiment, first copper forming a copper pillar is disposed and then the solder.

The detector substrate is disposed, e.g. in a direct placing or in a flip chip process. With the help of a reflow process, the solder forms a bump or ball and forms an electrically conductive interconnection to the charge collector contact. In the alternative embodiment using copper pillars, the solder bump forms on top of the copper pillar like a bump or a cap-like structure which forms an electrically conductive interconnection to the charge collector contact. The charge collector contact and the readout contact are bonded and an electrically conductive interconnection of corresponding charge collector contacts and readout contacts is provided. Due to the use of the SnBiAg solder, the product made by the disclosed method for producing a radiation detector device benefits from the advantages given for the radiation detector device like an improved thermo-mechanical stability over other solders like SnBi and a longer lifetime of the radiation detector device.

According to an aspect of the present invention, the detector substrate further comprises Tellurium.

The detector substrate comprises Tellurium in addition to Cadmium which results for example in CdTe as detector substrate which is beneficial for the detection of X-ray or gamma ray photons in imaging applications, in particular medical imaging applications.

According to an aspect of the present invention, the detector substrate further comprises Zinc.

The detector substrate comprises Zinc in addition to Cadmium which results for example in CdZnTe (CZT) as detector substrate which is beneficial for the detection of X-ray or gamma ray photons in imaging applications, in particular medical imaging applications.

According to an aspect of the present invention, the solder consists of Bismuth, Tin and Silver. According to this aspect, the solder is a ternary alloy. The solder consisting of Bismuth, Tin and Silver may contain trace metal impurities of further metals. The inclusion of Silver in the solder results in similar material properties like density, coefficient of thermal expansion and melting temperature, but at the same time an improvement of thermal conductivity and mechanical and thermo-mechanical reliability of the solder connection or solder joints is obtained. Therefore a longer lifetime of the radiation detector device may be achieved. Additional trace metal impurities have a negligible influence on the physical properties of the solder. Typically trace metal impurities are present with a concentration of less than 0.01 percent by weight.

According to an aspect of the present invention, the solder comprises Bismuth ranging between 54 percent and 60 percent by weight and Silver ranging between 0.01 percent and 2 percent by weight.

The eutectic ternary alloy SnBiAg consists approximately of 42 to 43 percent by weight of Tin, 56 to 57 percent of weight of Bismuth and 1 percent by weight of Silver. The above given ranges improve the thermo-mechanical properties of the solder due to the addition of Silver. As being close to the eutectic composition, a low melting temperature is granted.

According to an aspect of the present invention, the solder comprises Silver ranging between 0.05 and 0.50 percent by weight.

In order to achieve the most suitable properties of the solder, the solder comprises Silver ranging preferably between 0.01 and 1.0 percent by weight, more preferably between 0.05 and 0.75 percent by weight and even more preferably between 0.05 and 0.50 percent by weight.

According to an aspect of the present invention, the solder is lead-free. For environmental reasons, it is advantageous to use lead free materials. The radiation detector device according to this aspect of the invention comprises a solder which is lead free while fulfilling all requirements with regard to thermal conductivity and mechanical and thermo-mechanical reliability of the solder connection or solder joints.

According to an aspect of the present invention, the solder has a melting point ranging between 130°C and 150°C.
The solder has a melting point between 130°C and 150°C, preferably between 137°C and 140°C. The melting temperature range is favorable for the use of detector substrates comprising Cadmium, like CdTe and CdZnTe (CZT) which are temperature sensitive. The melting temperature range is also favorable for the steps of producing said radiation detector device.

According to an aspect of the present invention, the solder has a thermal conductivity of more than 21.6 W/mK.

The direct conversion of X-rays or gamma rays produces significant amount of heat in materials like CdTe and CdZnTe (CZT). The heat in the detector substrate has to be evacuated so that the temperature remains constant. The heat can be transferred from the converter material through the connection to the readout substrate and towards other electronic components of the detector. The improved thermal conductivity of the SnBiAg alloy leads to an improved heat transfer away from the detector substrate.

According to an aspect of the present invention, the solder has a grain size reduced by at least 20 percent compared to a binary eutectic alloy of Bismuth and Tin.

The grain size of the solder solidification microstructure grains decreases due to the addition of Silver in the alloy. The effective grain size is reduced by at least 20 percent, preferably at least 50 percent, and even more preferably by at least 100 percent by the Ag addition.

According to an aspect of the present invention, the solder has an increased tensile strength by at least 5 percent compared to the binary eutectic alloy of Bismuth and Tin.

The tensile strength increases due to the addition of Silver by at least 5 percent compared to the binary eutectic alloy of Bismuth and Tin, preferably by at least 20 percent compared to the binary eutectic alloy of Bismuth and Tin. The addition of silver leads to an improvement of the ductility of the alloy which results in an improvement of the thermal cycling performance. The improvement of the ductility is caused by the refinement of the solder solidification microstructure grains due to the addition of Silver. The Silver containing alloy is superior to the eutectic binary SnBi alloy with respect to survivability on typical shocks and sudden strains during transport, installation, and operation, as e.g. found in medical X-ray devices, e.g. C-arm or CT scanner devices. The ductility is improved over the binary SnBi alloy, especially at high deformation rates.

According to an aspect of the present invention, the medical imaging device is a CT scanner.

A CT scanner has strong requirements on the components due to the rotation of the components within the gantry and the high power consumption leading to the heating of the components like the X-ray tube, the radiation detector device, the electronics and others. Regarding the radiation detector device, the SnBiAg solder leads to an improved thermal conductivity, improved tensile strength, improved properties regarding the thermal cycling of the solder connection over other solders like SnBi and the solder may be used in combination with a detector substrate comprising Cadmium. This results in an advantageous, improved reliability of the solder connection and therefore the radiation detector device. The radiation detector device allows the use in a CT scanner.

Further objects and advantages of the present invention will be apparent from the following description of the preferred embodiments of the invention as illustrated in the accompanying drawings, in which:
FIG. 1 is an exemplary illustration of a radiation detector device;
FIG. 2 is an exemplary illustration of a radiation detector device comprising copper pillars;
FIG. 3 is an exemplary illustration of a CT scanner comprising a radiation detector device; and
FIG. 4 is a flowchart describing steps to be performed for producing a radiation detector device.

The present invention will be further described in detail in conjunction with the accompanying drawings and embodiments. It should be understood that the particular embodiments described herein are only used to illustrate the present invention but not to limit the present invention.

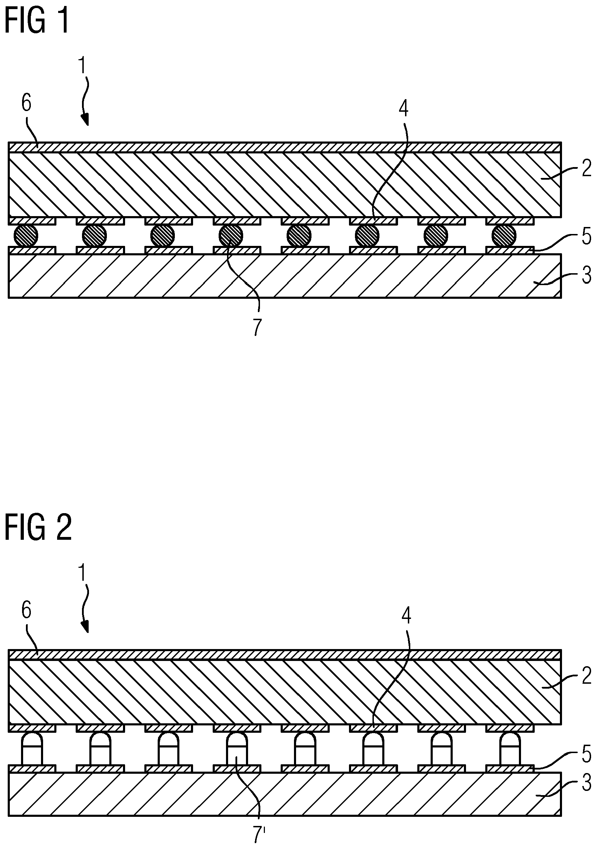

Figure 1 and Figure 2 show schematic drawings of a side view of the radiation detector device 1.

The radiation detector device 1 comprises at least one detection unit or pixel which comprises a volume of the detector substrate 2 wherein the volume is defined by the electric field of a bias voltage, the charge collector contact 4, the connection 7, 7' between the charge collector contact 4 and the readout contact 5, the readout contact 5 and the readout substrate 3 with analog and digital circuits for signal processing and control. In the example, the pixelated structure of the radiation detector device 1 comprises eight pixels in the cross section, e.g. as row. Perpendicular, a plurality of pixels may represent the columns of the pixel matrix (not shown).

The radiation detector device 1 may be embodied as a hybrid assembly comprising a single detector substrate 2 and a single readout substrate 3 as shown in Figure 1 and 2. In a further embodiment (not illustrated), the radiation detector device may be constructed as a hybrid assembly comprising a plurality of detector substrates 2 and/or readout substrates 3.

In operation, the detector substrate 2 is facing a radiation source (not shown in Figs. 1 and 2). The detector substrate 2 comprises a charge biasing electrode 6 on the front side of the detector substrate 2. The detector substrate 2 further comprises a charge collector contact 4 which is part of the charge collector electrode which is located at the back side of the detector substrate 2. An electric biasing field is applied between the readout contact 5 and the charge biasing electrode 6 in order to separate charges induced by energy deposition in the detector substrate 2, e.g. by a photon.

The detector substrate 2 comprises a semiconductor material which contains Cadmium, e.g. CdTe or CdZnTe (CZT).

The readout substrate 3 has a readout contact 5, e.g. a standard metallization readout contact (Under Bump Metallization, UBM, Under Bump Metallurgy). The readout substrate 3 provides analog and digital processing of the signals as well as means for controlling the radiation detector device 1, e.g. counting mode and setting thresholds. The processing circuits are embodied as an ASIC (Application Specific Integrated Circuit). The resulting signals are led to further peripheral data processing units. The radiation detector device 1 is controlled by a peripheral controller unit.

The charge collector contact 4 and the readout contact 5 may be embodied as a standard metallization contact (Under Bump Metallization, UBM, Under Bump Metallurgy). The charge collector contact 4 may further comprise a passivation opening in the detector substrate 2. The readout contact 5 may further comprise a passivation opening in the readout substrate 3.

The connection 7, 7' between the detector substrate 2 and the readout substrate 3 comprises a solder connection. The solder connection comprises a solder. The solder comprises Tin, Bismuth and Silver. The solder can be used as prefabricated alloy, for example in the form of solder ball or solder paste. In a further embodiment, the solder material can also be used in combination with copper pillars. The connection 7 may be embodied comprising solder bumps to form solder balls as illustrated in Figure 1 or, in an alternative embodiment, the connection 7' may be embodied comprising solder bumps or caps in combination with copper pillars as illustrated in Figure 2. The connection 7, 7' may further comprise an underfill between the detector substrate 2 and the readout substrate 3. Besides of providing electrical conductive connection between the detector substrate 2 and the readout substrate 3, the connection 7, 7' serves as transport of heat from the detector substrate 2 towards the readout substrate 3 and other components.

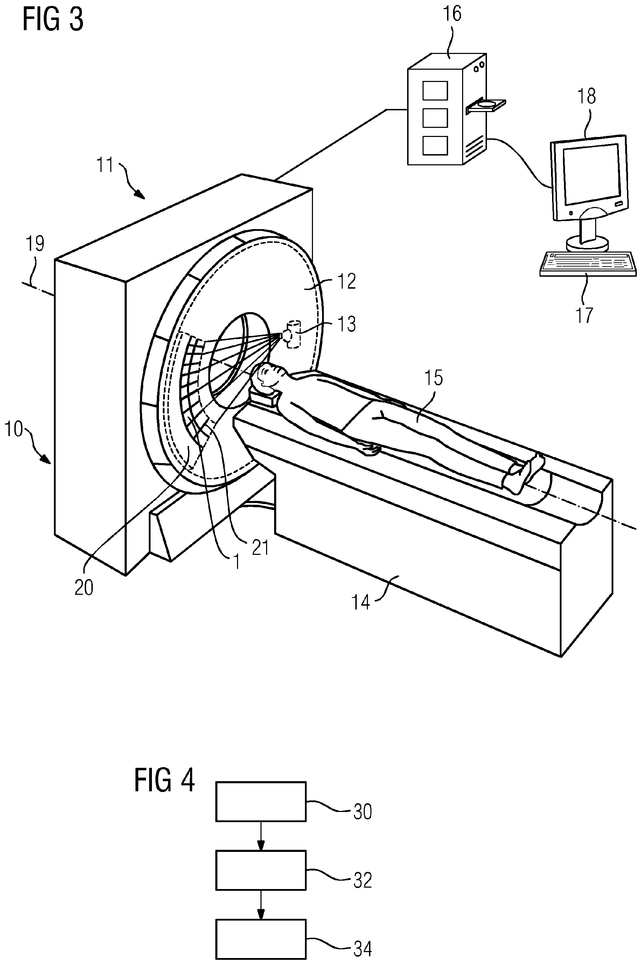

In Figure 3, a CT scanner 10 is shown. The CT scanner 10 comprises a gantry 11 with a rotor 12. The rotor comprises the radiation source 13 and the detector 20. In a conventional CT scanner 10, the radiation source 13 is embodied as an X-ray tube which emits X-rays. The detector 20 comprises a plurality of radiation detector devices 1. Scattered radiation grids 21 are attached to the radiation detector devices 1. As shown, the detector 20 may be curved. The radiation detector devices 1 are aligned facing the radiation source and the scattered radiation grids 21 are aligned to the X-rays emitted by the radiation source 13. A patient 15 is positioned on a patient couch 14. The patient 15 can be moved along the axis of rotation 19 through the gantry 11. For controlling and the calculation of e.g. sectional images, a calculator unit 16 is used. An input device 17 and an output device 18 are connected to the calculator unit 16.

In Figure 4, a method for producing a radiation detector device is illustrated by a flowchart. The method comprises the steps of providing a readout substrate 30, applying a solder connection 32 and disposing the detector substrate 34.

The readout substrate 3 is provided in step 30. To be prepared for the application of the solder connection, a readout contact 5, e.g. a standard metallization readout contact (UBM), is provided. Further steps may be comprised like providing a passivation opening.

In the next step, the solder connection is applied. Therefore, a solder ball and solder flux are disposed at the readout contact 5. In an alternative embodiment, a solder paste is disposed at the readout contact 5. Under a reflow process a solder bump is formed which is connected to the readout contact 5.

The solder comprises Bismuth, Tin and Silver. The amount of Silver ranges between 0.01 percent and 2 percent by weight. The amount of Bismuth ranges between 54 percent and 60 percent by weight. A solder of this composition has a melting range of about 135 to 145 °C.

The detector substrate 2 is disposed, e.g. in a direct placing or in a flip chip process. A solder flux is disposed between the charge collector contact 4 and the solder bump. With the help of a reflow process with temperatures below 180°C, the solder bump forms an electrically conductive interconnection to the charge collector contact 4. In an alternative embodiment using copper pillars, the bump forms on top of a copper pillar like a bump or a cap-like structure which forms an electrically conductive interconnection to the charge collector contact 4. The charge collector contact 4 and the readout contact 5 are bonded and an electrically conductive interconnection of corresponding charge collector contact 4 and readout contact 5 is provided. The connection 7, 7' is installed.
Within the production of the radiation detector device several cleaning steps may be included.

## Claims

1. A radiation detector device comprising:
- a detector substrate (2) for direct conversion of X-ray and/or gamma quanta to electrical charge, comprising a semiconductor material comprising Cadmium;
- a readout substrate (5) having a readout contact;
- a charge collector contact (4) for collecting an electrical charge from said detector substrate;
- a connection (7, 7') wherein the connection electrically interconnects the charge collector contact and the readout contact;
- wherein the connection comprises a solder connection **characterised in that** the solder comprises Bismuth, Tin and Silver.

2. A radiation detector device of claim 1, wherein the detector substrate further comprises Tellurium.

3. A radiation detector device as claimed in one of the preceding claims, wherein the detector substrate further comprises Zinc.

4. A radiation detector device as claimed in one of the preceding claims, wherein the solder consists of Bismuth, Tin and Silver.

5. A radiation detector device as claimed in one of the preceding claims, wherein the solder comprises Bismuth ranging between 54 percent and 60 percent by weight and Silver ranging between 0.01 percent and 2 percent by weight.

6. A radiation detector device as claimed in one of the preceding claims, wherein the solder comprises Silver ranging between 0.05 and 0.50 percent by weight.

7. A radiation detector device as claimed in one of the preceding claims, wherein the solder is lead-free.

8. A radiation detector device as claimed in one of the preceding claims, wherein the solder has a melting point ranging between 130°C and 150°C.

9. A radiation detector device as claimed in one of the preceding claims, wherein the solder has a thermal conductivity of more than 21.6 W/mK.

10. A radiation detector device as claimed in one of the preceding claims, wherein the solder has a grain size reduced by at least 20 percent compared to a binary eutectic alloy of Bismuth and Tin.

11. A radiation detector device as claimed in one of the preceding claims, wherein the solder has an increased tensile strength by at least 5 percent compared to the binary eutectic alloy of Bismuth and Tin.

12. A medical imaging device comprising a radiation detector device as claimed in one of the preceding claims.

13. A medical imaging device as claimed in claim 12, wherein the medical imaging device is a CT scanner.

14. A method for producing a radiation detector device as claimed in one of the preceding claims 1 to 11 comprising the steps of:
a) Providing a readout substrate having a readout contact;
b) Applying a solder connection containing a solder comprising Bismuth, Tin and Silver;
c) Disposing the detector substrate to bond a charge collector contact and the readout contact by a solder reflow technique to form an electrically conductive interconnection of corresponding charge collector contact and readout contact.

## Patentansprüche

1. Strahlungsdetektorvorrichtung, umfassend:
- ein Detektorsubstrat (2) für eine direkte Wandlung von Röntgenstrahlung und/oder Gammaquanten in eine elektrische Ladung, das ein Halbleitermaterial umfasst, das seinerseits Cadmium umfasst;
- ein Auslesesubstrat (5), das einen Auslesekontakt aufweist;
- einen Ladungssammlerkontakt (4) zum Sammeln einer elektrischen Ladung von dem Detektorsubstrat;
- eine Verbindung (7, 7'), wobei die Verbindung den Ladungssammlerkontakt und den Auslesekontakt elektrisch verbindet;
- wobei die Verbindung eine Lötverbindung umfasst, **dadurch gekennzeichnet, dass** die Lötverbindung Wismut, Zinn und Silber umfasst.

2. Strahlungsdetektorvorrichtung nach Anspruch 1, wobei das Detektorsubstrat außerdem Tellur umfasst.

3. Strahlungsdetektorvorrichtung nach einem der vorhergehenden Ansprüche, wobei das Detektorsubstrat außerdem Zink umfasst.

4. Strahlungsdetektorvorrichtung nach einem der vorhergehenden Ansprüche, wobei das Lötmittel aus Wismut, Zinn und Silber besteht.

5. Strahlungsdetektorvorrichtung nach einem der vorhergehenden Ansprüche, wobei das Lötmittel Wismut in einem Bereich zwischen 54 Gew.-% und 60 Gew.-% und Silber in einem Bereich zwischen 0,01 Gew.-% und 2 Gew.-% umfasst.

6. Strahlungsdetektorvorrichtung nach einem der vorhergehenden Ansprüche, wobei das Lötmittel Silber in einem Bereich zwischen 0,05 Gew.-% und 0,50 Gew.-% umfasst.

7. Strahlungsdetektorvorrichtung nach einem der vorhergehenden Ansprüche, wobei das Lötmittel bleifrei ist.

8. Strahlungsdetektorvorrichtung nach einem der vorhergehenden Ansprüche, wobei das Lötmittel einen Schmelzpunkt in einem Bereich zwischen 130°C und 150°C aufweist.

9. Strahlungsdetektorvorrichtung nach einem der vorhergehenden Ansprüche, wobei das Lötmittel eine Wärmeleitfähigkeit von mehr als 21,6 W/mK aufweist.

10. Strahlungsdetektorvorrichtung nach einem der vorhergehenden Ansprüche, wobei das Lötmittel eine Korngröße aufweist, die um mindestens 20 Prozent im Vergleich zu einer binären eutektischen Legierung aus Wismut und Zinn verringert ist.

11. Strahlungsdetektorvorrichtung nach einem der vorhergehenden Ansprüche, wobei das Lötmittel eine Zugfestigkeit aufweist, die um mindestens 5 Prozent im Vergleich zu der binären eutektischen Legierung aus Wismut und Zinn vergrößert ist.

12. Medizinische Bilderfassungseinheit die eine Strahlungsdetektorvorrichtung nach einem der vorhergehenden Ansprüche umfasst.

13. Medizinische Bilderfassungseinheit nach Anspruch 12, wobei die medizinische Bilderfassungseinheit ein CT-Scanner ist.

14. Verfahren zum Herstellen einer Strahlungsdetektorvorrichtung nach einem der Ansprüche 1 bis 11, das die folgenden Schritte umfasst:
a) Bereitstellen eines Auslesesubstrats, das einen Auslesekontakt aufweist;
b) Anwenden einer Lötverbindung, die ein Lötmittel enthält, das Wismut, Zinn und Silber umfasst;
c) Anordnen des Detektorsubstrats, um einen Ladungssammlerkontakt und den Auslesekontakt mithilfe einer Aufschmelzlöttechnik so zu bonden, dass eine elektrisch leitfähige Verbindung des entsprechenden Ladungssammlerkontakts und des Auslesekontakts gebildet wird.

## Revendications

1. Dispositif de détection d'un rayonnement comprenant :
- un substrat (2) formant détecteur pour transformer directement des quanta de rayons X et/ou gamma en charge électrique, comprenant un matériau semi-conducteur comprenant du cadmium;
- un substrat (5) de lecture ayant un contact de lecture;
- un contact (4) formant collecteur de charge pour collecter une charge électrique du substrat formant détecteur;
- une connexion (7, 7'), la connexion interconnectant électriquement le contact formant collecteur de charge et le contact de lecture;
- dans lequel la connexion comprend une connexion brasée;
**caractérisé en ce que** la brasure comprend du bismuth, de l'étain et de l'argent.

2. Dispositif de détection d'un rayonnement suivant la revendication 1, dans lequel le substrat formant conducteur comprend, en outre, du tellure.

3. Dispositif de détection d'un rayonnement suivant l'une des revendications précédentes, dans lequel le substrat formant détecteur comprend, en outre, du zinc.

4. Dispositif de détection d'un rayonnement suivant l'une des revendications précédentes, dans lequel la brasure consiste en du bismuth, de l'étain et de l'argent.

5. Dispositif de détection d'un rayonnement suivant l'une des revendications précédentes, dans lequel la brasure comprend du bismuth en un pourcentage compris entre 54 pourcent et 60 pourcent en poids et de l'argent en un pourcentage compris entre 0,001 pourcent et 2 pourcent en poids.

6. Dispositif de détection d'un rayonnement suivant l'une des revendications précédentes, dans lequel la brasure comprend de l'argent en un pourcentage compris entre 0,05 et 0,50 pourcent en poids.

7. Dispositif de détection d'un rayonnement suivant l'une des revendications précédentes, dans lequel la brasure est exempte de plomb.

8. Dispositif de détection d'un rayonnement suivant l'une des revendications précédentes, dans lequel la brasure a un point de fusion compris entre 130°C et 150°C.

9. Dispositif de détection d'un rayonnement suivant l'une des revendications précédentes, dans lequel la brasure a une conductivité thermique supérieure à 21,6 W/mK.

10. Dispositif de détection d'un rayonnement suivant l'une des revendications précédentes, dans lequel la brasure a une granulométrie réduite d'au moins 20 pourcent par rapport à un alliage eutectique binaire de bismuth et d'étain.

11. Dispositif de détection d'un rayonnement suivant l'une des revendications précédentes, dans lequel la brasure a une résistance à la traction accrue d'au moins 5 pourcent par rapport à l'alliage eutectique binaire de bismuth et d'étain.

12. Dispositif d'imagerie médicale comprenant un dispositif de détection d'un rayonnement, tel que revendiqué à l'une des revendications précédentes.

13. Dispositif d'imagerie médicale, tel que revendiqué à la revendication 12, dans lequel le dispositif d'imagerie médicale est un scanner CT.

14. Procédé de production d'un dispositif de détection d'un rayonnement, tel que revendiqué à l'une des revendications précédentes 1 à 11, comprenant les stades dans lesquels :
a) on se procure un substrat de lecture ayant un contact de lecture;
b) on applique une connexion brasée contenant une brasure comprenant du bismuth, de l'étain et de l'argent;
c) on met le substrat formant détecteur, de manière à lier un contact formant collecteur de charge et le contact de lecture par une technique de fusion de brasure pour former une interconnexion conductrice de l'électricité du contact de collecteur de charge correspondant et du contact de lecture.
